(19) Europäisches Patentamt
European Patent Office
Office européen des brevets

(11) Publication number: **0 248 434 B1**

(12) **EUROPEAN PATENT SPECIFICATION**

(45) Date of publication of patent specification: **11.09.91**

(51) Int. Cl.[5]: **C07D 285/08**, //C07D501/46

(21) Application number: **87108124.6**

(22) Date of filing: **04.06.87**

The file contains technical information submitted after the application was filed and not included in this specification

# (54) Thiadiazolylacetamide derivatives, process for the preparation thereof and their use.

(30) Priority: **05.06.86 JP 129233/86**

(43) Date of publication of application:
**09.12.87 Bulletin 87/50**

(45) Publication of the grant of the patent:
**11.09.91 Bulletin 91/37**

(84) Designated Contracting States:
**AT BE CH DE ES FR GB GR IT LI LU NL SE**

(56) References cited:
**FR-A- 2 564 840**
**GB-A- 2 094 794**
**US-A- 4 406 898**

**JOURNAL OF ORGANIC CHEMISTRY, vol. 34, no. 11, November 1969, pages 3635-3638, Easton, PA, US; B.C. TYSON et al.: "Chemistry of 4-pyridineglyoxylonitrile oxime and methyl 4-pyridineglyoxylate oxime ethers"**

(73) Proprietor: **Eisai Co., Ltd.**
**6-10, Koishikawa 4-chome Bunkyo-ku**
**Tokyo 112(JP)**

(72) Inventor: **Kanai, Takeo**
**4680-103 Kyuchu Kashima-cho**
**Kashima-gun Ibaraki Prefecture(JP)**
Inventor: **Takayanagi, Keizo**
**2333-249 Kyuchu Kashima-cho**
**Kashima-gun Ibaraki Prefecture(JP)**
Inventor: **Tanaka, Yasuhide**
**465-4 Komoda Misato-machi**
**Kodama-gun Saitama Prefecture(JP)**
Inventor: **Koiwa, Atsushi**
**10000-1481 Yatabe Hazaki-cho**
**Kashima-gun Ibaraki Prefecture(JP)**
Inventor: **Endoh, Shinichi**
**6-16-27 Shitte-chuo Kamisu-cho**
**Kashima-gun Ibaraki Prefecture(JP)**
Inventor: **Satoh, Katsuhiko**
**6-16-27 Shitte-chuo Kamisu-cho**
**Kashima-gun Ibaraki Prefecture(JP)**
Inventor: **Nomoto, Seiichiro**
**1-134-2 Kariya Ushiku-machi**
**Inashiki-gun Ibaraki Prefecture(JP)**

**EP 0 248 434 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Sugiyama, Isao**
**29-4 Higashi-arai Yatabe-machi**
**Tsukuba-gun Ibaraki Prefecture(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

## Description

BACKGROUND OF THE INVENTION

1) Field of the Invention:

This invention relates to thiadiazolylacetamide derivatives useful as intermediates for antibacterial agents and a process for the preparation of same.

2) Description of the Background:

Japanese Patent Application Laid-open No.158769/1982, GB-A-2094794, disclose a process for the preparation of thiadiazolylacetic acid derivatives which are useful as acylating agents upon preparation of 7-acylamino-cephalosporin compounds. According to the above process, the corresponding acetonitrile derivatives are hydrolyzed in the presence of a base. The above process however results in the formation of byproducts so that the yields are very low, i.e., 30% or lower. The above process is hence not considered to be satisfactory from the industrial viewpoint.

SUMMARY OF THE INVENTION

With the foregoing in view, the present inventors have carried out an investigation in order to develop an industrial process for the preparation of thiadiazolylacetic acid derivatives.

Accordingly, an object of this invention is to provide novel thiadiazolylacetamide derivatives useful as intermediates for antibacterial agents.

Another object of this invention is to provide a process for the preparation of such novel thiadiazolylactamide derivatives.

In one aspect of this invention, there is thus provided a thiadiazolylacetamide derivative represented by the following formula (I);

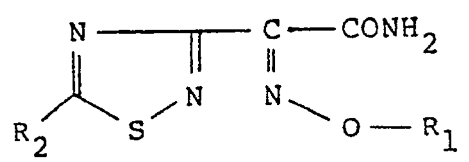

(I)

wherein $R_1$ means a methyl group and $R_2$ denotes an amino group which may optionally be blocked by a protecting group, or a salt thereof.

In another aspect of this invention, there is also provided a process for the preparation of the thiadiazolylacetamide derivative represented by the formula (I) or a salt thereof, which comprises reacting a compound represented by the following formula (II):

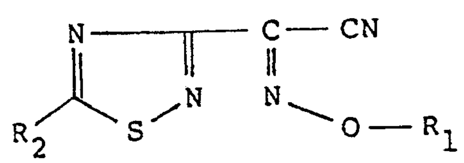

(II)

wherein $R_1$ and $R_2$ have the same meaning as defined above, or a salt thereof with an oxidizing agent and a base, and then removing the protecting group, if necessary.

By subjecting the thiadiazolylacetamide derivative of the formula (I) to hydrolysis in the presence of a base similar to that employed above upon its preparation, the corresponding thiadiazolylacetic acid derivative can be obtained in a high yield. This preparation process of the thiadiazolylacetic acid derivative is a two-step reaction in which the thiadiazolylacetamide derivative (I) is first obtained from the thiadiazolylacetonitrile derivative (II) and the thiadiazolylacetic acid derivative is then obtained from the thiadiazolylacetamide derivative (I). The two-step reaction however enjoys a higher yield and is hence an industrially-superior process, compared with the above-described conventional process in which the

thiadiazolylacetic acid derivative is obtained directly from the corresponding acetonitrile derivative. The intermediate for the above two-step reaction, namely, the thiadiazolylacetamide derivative of this invention is a very useful compound.

The above and other objects, features and advantages of the present invention will become apparent from the following description and the appended claims.

## DETAILED DESCRIPTION OF THE INVENTION AND PREFERRED EMBODIMENTS

Regarding the kind of a protecting group usable to form the "amino group blocked by a protecting group" represented by $R_2$, those known generally as protecting groups for amino groups may be employed. As exemplary protecting groups, may be mentioned substituted or unsubstituted lower alkanoyl groups such as formyl, acetyl, chloroacetyl, dichloroacetyl, propionyl and phenoxyacetyl; substituted or unsubstituted lower alkoxycarbonyl groups such as benzyloxycarbonyl, t-butoxycarbonyl and p-nitrobenzyloxycarbonyl; substituted lower alkyl groups such as trityl, p-methoxybenzyl and diphenylmethyl; substituted silyl groups such as trimethylsilyl and t-butyldimethylsilyl; and substituted and unsubstituted benzylidene groups such as benzylidene, salicylidene, 3,5-di(t-butyl)-4-hydroxybenzylidene and 3,5-di(t-butyl)benzylidene.

On the other hand, illustrative examples of the salt of the compound of the formula (I) may include inorganic acid salts such as hydrochloride, sulfate, carbonate, bicarbonate, hydrobromide and hydroiodide; organic carboxylates such as acetate, maleate, lactate, tartrate and trifluoroacetate; organic sulfonates such as methanesulfonate, benzenesulfonate and toluenesulfonate; amino acid salts such as aspartate and glutamate.

The thiadiazolylacetamide derivative of the formula (I) can be prepared by the above-described process. As the salt of the compound of the general formula (II), may be employed the same salt as mentioned above with respect to the salt of the compound of the formula (I).

The above reaction can be conducted in an aqueous solvent, preferably, at a reaction temperature of 0°C - 70°C, more preferably, 15°C - 50°C. The removal of the protecting group may be effected in a usual manner, for example, by hydrolysis or reduction in accordance with the kind of the protecting group.

Hydrogen peroxide or oxygen may be used as the oxidizing agent. As an alternative, it is also possible to adopt the electrolytic oxidation method in which oxygen is produced in situ.

As exemplary bases useful in the practice of the above process, there may be mentioned sodium hydroxide, potassium hydroxide, sodium carbonate, potassium carbonate, sodium hydrogencarbonate, potassium hydrogencarbonate, sodium acetate or potassium acetate.

The process is also carried out in an aqueous solvent.

As the aqueous solvent, it is possible to use water, a buffer, or a mixed solvent of water or a buffer and a lower alcohol such as methanol or ethanol.

The present invention will hereinafter be described in further detail by the following Examples and Experiments:

Example 1:

2-(5-Amino-1,2,4-thiadiazol-3-yl)-(Z)-2-methoxyiminoacetamide

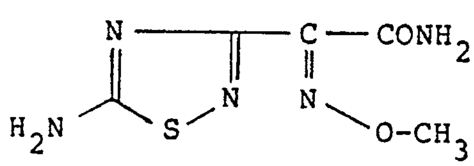

To a liquid mixture of 400 mℓ of water, 10 g of sodium hydroxide and 160 mℓ of a 35% aqueous hydrogen peroxide solution, was added 80 g of 2-(5-amino-1,2,4-thiadiazol-3-yl)-(Z)-2-methoxyiminoacetonitrile. The contents were stirred under ice-cooling for 5 hours. The resultant deposit was collected by filtration, washed successively with water and acetone, and then dried to obtain 77.7 g of the desired product (yield: 88.4%).

Example 2:

2-5-Chloroacetamido-1,2,4-thiadiazol-3-yl-(Z)-2-methoxyiminoacetamide

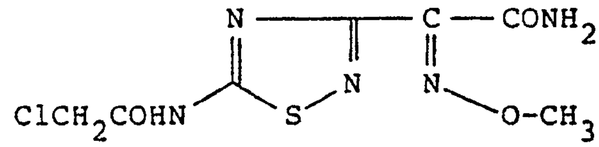

To a liquid mixture of 100 mℓ of water, 2.0 g of sodium hydroxide and 10.0 mℓ of a 35% aqueous hydrogen peroxide solution, 5.0 g of 2-(5-chloro-acetamido-1,2,,4-thiadiazol-3-yl)-(Z)-2-methoxyimino-acetonitrile was added. The contents were stirred for 30 minutes. The reaction mixture was cooled down to -2° C and the resultant deposit was collected by filtration. The deposit was successively treated with ice water and ethyl ether, and then dried to obtain 2.0 g of the desired product.

In addition, the above filtrate was extracted with ethyl acetate. After drying the extract by adding anhydrous magnesium sulfate to same, the ethyl acetate was distilled off. Isopropyl ether was added to the residue to crystallize the same, thereby obtaining 2.2 g of the desired product as a second crop.

The desired product was hence obtained in a total amount of 4.2 g (yield: 78.6%).

In order to demonstrate further advantageous effects of the present invention, a description will next be made of an experiment on the preparation of a thiadiazolylacetic acid derivative, which is known as an intermediate for an antibacterial agent, by using a compound of this invention as well as another experiment on the preparation of the same compound by a conventional process.

Experiment 1: (Process using a compound of the invention)

2-(5-Amino-1,2,4-thiadiazol-3-yl)-(Z)-2-methoxyiminoacetic acid

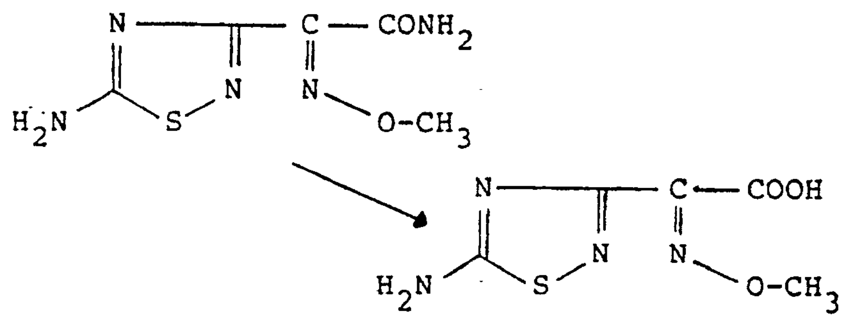

A mixture of 80 mℓ of a 2N aqueous solution of sodium hydroxide and 4.02 g of 2-(5-amino-1,2,4-thiadiazol-3-yl)-(Z)-2-methoxyiminoacetamide was stirred at 50° C for 5 hours. The reaction mixture was cooled down to 10° C and was then adjusted with concentrated hydrochloric acid to pH 1. The reaction mixture was thereafter extracted with ethyl acetate and anhydrous magnesium sulfate was added to the resultant extract to dry same. The ethyl acetate solution was then concentrated under reduced pressure, followed by a dropwise addition of 100 mℓ of isopropyl ether. Insoluble matter was collected by filtration, washed with isopropyl ether, and then dried to obtain 3.2 g of the desired product as white powder (yield: 79.2%). Its purity was found to be 98.2% as a result of an HPLC analysis.

Experiment 2: (Conventional process)

2-(5-Amino-1,2,4-thiadiazol-3-yl)-(Z)-2-methoxyiminoacetic acid

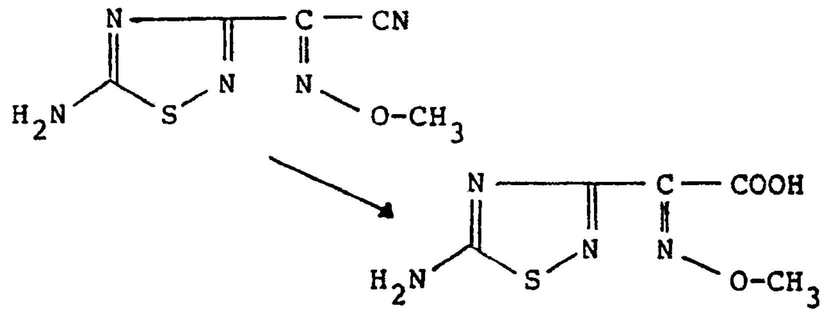

To a suspension of 7.50 g of 2-(5-amino-1,2,4-thiadiazol-3-yl)-(Z)-2-methoxyiminoacetonitrile and 40 mℓ of water, 95 mℓ of a 4N aqueous solution of sodium hydroxide was added dropwise at 40° C. The contents were stirred for 5 hours at 50° C - 55° C. After cooling the reaction mixture to 10° C, 6N hydrochloric acid was added dropwise to adjust the pH of the reaction mixture to 2.4. Thereafter, 50 mℓ of ethyl acetate was added to the reaction mixture to extract and remove impurities. After adjusting the pH of the water layer to pH 0.8 with 6N hydrochloric acid, 0.5 g of activated carbon was added and the resultant mixture was filtered. The filtrate was extracted with ethyl acetate and anhydrous magnesium sulfate was added to the extract to dry same. The ethyl acetate was distilled off under reduced pressure and 100 mℓ of isopropyl ether was added to the residue.

The resulting deposit was collected by filtration and was then dried, thereby obtaining 3.5 g of a crude product as pale yellow powder [purity: 79.5% (by HPLC analysis)].

Seventy milliliters (70 mℓ) of a 1:1 mixture of ethyl acetate and methyl ethyl ketone was then added to 3.5 g of the crude product and the resultant mixture was heated for 15 minutes at 45° C - 50° C. The reaction mixture was filtered at the same temperature to collect the resultant precipitate. The thus-collected precipitate was washed with 7 mℓ of ethyl acetate and was then dried to obtain 1.6 g of the desired product as white powder (yield: 19.7%). Its purity was found to be 97.5% as a result of an HPLC analysis.

Table: Data of Physical Properties

| Compound | Melting point (°C) | IR spectrum ($cm^{-1}$, Nujol) | NMR spectrum (δ, DMSO-$d_6$) |
|---|---|---|---|
| Example 1 | 245 - 252 (decomposed) | 1685 | 3.89(3H,s), 7.58(1H,s), 7.86(1H,s), 8.09(2H,s) |
| Example 2 | 247 - 250 (decomposed) | 1680 | 1.39(3H,t,J=7Hz), 4.16(2H,q,J=7Hz), 7.56(1H,s), 7.85(1H,s), 8.10(2H,s) |
| Example ~~3~~ 2 | 238 - 252 (decomposed) | 1686 | 3.89(3H,s), 4.19(2H,s), 7.49(1H,s), 7.81(1H,s) |
| Experiment 1 | 178 - 179 (decomposed) | 1720 | 3.95(3H,s), 8.19(2H,s) |
| Experiment 2 | 175 - 182 (decomposed) | 1720 | 3.95(3H,s), 8.19(2H,s) |

## Claims

**Claims for the following Contracting States : BE, CH, DE, FR, GB, GR, IT, LI, LU, NL, SE**

**1.** A thiadiazolylacetamide derivative represented by the following formula (I):

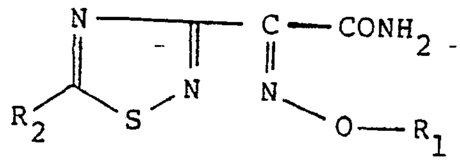

(I)

wherein $R_1$ means a methyl group and $R_2$ denotes an amino group which may optionally be blocked by a protecting group, or a salt thereof.

**2.** The compound as claimed in claim 1, which is 2-(5-amino-1,2,4-thiadiazol-3-yl)-(Z)-2-methoyiminoacetamide or a salt thereof.

**3.** The compound as claimed in claim 1, which is 2-(5-chloroacetamido-1,2,4-thiadiazol-3-yl)-(Z)-2-methoxyimino-acetamide or a salt thereof.

**4.** A process for the preparation of a thiadiazolylacetamide derivative represented by the following formula (I):

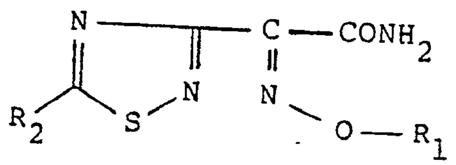

(I)

wherein $R_1$ means a methyl group and $R_2$ denotes an amino group which may optionally be blocked by a protection group, or a salt thereof, which comprises reacting a compound represented by the following formula (II):

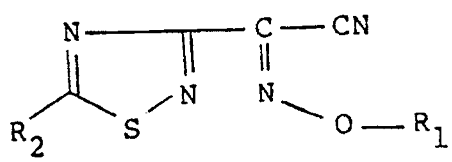

(II)

wherein $R_1$ and $R_2$ have the same meaning as defined above, or a salt thereof with an oxidizing agent and a base, and then removing the protecting group, if necessary.

**5.** The process as claimed in claim 4, wherein the reaction is effected at 0 °C - 70 °C.

**6.** The process as claimed in claim 5, wherein the reaction is effected at 15 °C - 50 °C.

**7.** The process as claimed in claim 4, wherein the oxidizing agent is hydrogen peroxide or oxygen.

**8.** The process as claimed in claim 4, wherein the base is sodium hydroxide, potassium hydroxide, sodium carbonate, potassium carbonate, sodium hydrogencarbonate, potassium hydrogencarbonate, sodium acetate or potassium acetate.

**9.** The process as claimed in claim 8, wherein the base is sodium hydroxide.

**10.** The process as claimed in claim 4, wherein the process is carried out in an aqueous solvent.

**11.** The process as claimed in claim 10, wherein the aqueous solvent is water, a buffer, or a mixed solvent of water or a buffer and a lower alcohol.

**12.** The process as claimed in claim 11, wherein the lower alcohol is methanol or ethanol.

13. The use of a thiadiazolylacetamide derivative of claim 1 as an intermediate for the making of antibacterial compositions.

**Claims for the following Contracting States : AT, ES**

1. A process for the preparation of a thiadiazolylacetamide derivative represented by the following formula (I):

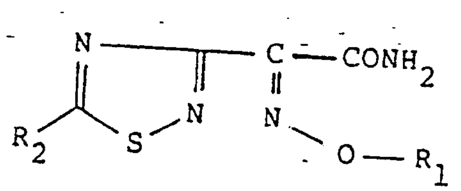

(I)

wherein $R_1$ means a methyl group and $R_2$ denotes an amino group which may optionally be blocked by a protecting group, or a salt thereof, which comprises reacting a compound represented by the following formula (II):

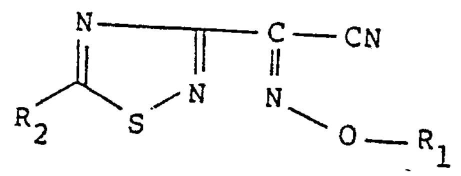

(II)

wherein $R_1$ and $R_1$ have the same meaning as defined above, or a salt thereof with an oxidising agent and a base, and then removing the protecting group, if necessary.

2. The process as claimed in claim 1, wherein the reaction is effected at 0° C - 70° C.

3. The process as claimed in claim 2, wherein the reaction is effected at 15° C - 50° C.

4. The process as claimed in Claim 1, wherein the oxidizing agent is hydrogen peroxide or oxygen.

5. The process as claimed in Claim 1, wherein the base is sodium hydroxide, potassium hydroxide, sodium carbonate, potassium carbonate, sodium hydrogencarbonate, potassium hydrogencarbonate, sodium acetate or potassium acetate.

6. The process as claimed in Claim 5, wherein the base is sodium hydroxide.

7. The process as claimed in Claim 2, wherein the process is carried out in an aqueous solvent.

8. The process as claimed in Claim 7, wherein the aqueous solvent is water, a buffer, or a mixed solvent of water or a buffer and a lower alcohol.

9. The process as claimed in Claim 8, wherein the lower alcohol is methanol or ethanol.

10. The use of a thiadiazolylacetamide derivative of claim 1 as an intermediate for the making of antibacterial compositions.

**Revendications**

**Revendications pour les Etats contractants suivants : BE, CH, DE, FR, GB, GR, IT, LI, LU, NL**

1. Dé'rivé de thiadiazolylacétamide représenté par la formule (I) suivante:

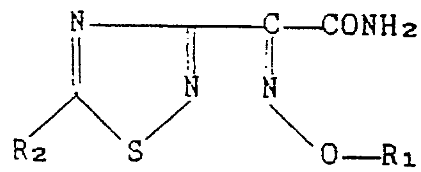

dans laquelle $R_1$ représente un groupe méthyle et $R_2$ désigne un groupe amino, facultativement bloqué par un groupe protecteur, ou un sel de celui-ci.

2. Composé tel que revendiqué dans la revendication 1, qui est le 2-(5-amino-1,2,4-thiadiazol-3-yl)-(Z)-2-méthoxyiminoacétamide ou un sel de celui-ci.

3. Composé tel que revendiqué dans la revendication 1, qui est le 2-(5-chloroacétamido-1,2,4-thiadiazol-3-yl)-(Z)-2-méthoxyiminoacétamide ou un sel de celui-ci.

4. Procédé pour préparer un dérivé de thiadiazolylacétamide représenté par la formule (I) suivante:

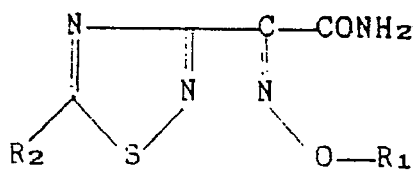

(I)

dans laquelle $R_1$ représente un groupe méthyle et $R_2$ désigne un groupe amino, facultativement bloqué par un groupe protecteur, ou un sel de celui-ci, qui comprend la réaction d'un composé représenté par la formule (II) suivante:

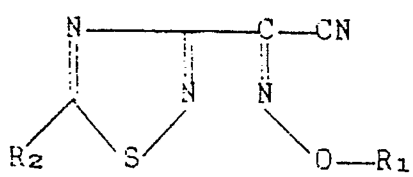

(II)

dans laquelle $R_1$ et $R_2$ ont la même signification que celle définie ci-dessus, ou un sel de celui-ci, avec un agent oxydant et une base, et ensuite l'élimination du groupe protecteur, si nécessaire.

5. Procédé tel que revendiqué dans la revendication 4, dans lequel la réaction est effectuée entre 0°C et 70°C.

6. Procédé tel que revendiqué dans la revendication 5, dans lequel la réaction est effectuée entre 15°C et 50°C.

7. Procédé tel que revendiqué dans la revendication 4, dans lequel l'agent oxydant est le peroxyde d'hydrogène ou l'oxygène.

8. Procédé tel que revendiqué dans la revendication 4, dans lequel la base est l'hydroxyde de sodium, l'hydroxyde de potassium, le carbonate de sodium, le carbonate de potassium, l'hydrogénocarbonate de sodium, l'hydrogénocarbonate de potassium, l'acétate de sodium ou l'acétate de potassium.

9. Procédé' tel que revendiqué dans la revendication 8, dans lequel la base est l'hydroxyde de sodium.

10. Procédé tel que revendiqué dans la revendication 4, dans lequel le procédé est effectué dans un solvant aqueux.

11. Procédé tel que revendiqué dans la revendication 10, dans lequel le solvant est l'eau, un tampon ou un solvant composé d'un mélange d'eau ou d'un tampon et d'un alcool inférieur.

12. Procédé tel que revendiqué dans la revendication 11, dans lequel l'alcool inférieur est le méthanol ou l'éthanol.

13. Utilisation du dérivé de thiadiazolylacétamide de la revendication 1, comme intermédiaire pour l'élaboration de compositions antibactériennes.

**Revendications pour les Etats contractants suivants : AT, ES**

1. Procédé pour préparer un dérivé de thiadiazolylacétamide représenté par la formule (I) suivante:

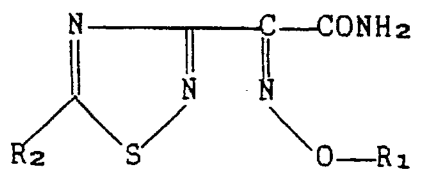

(I)

dans laquelle $R_1$ représente un groupe méthyle et $R_2$ désigne un groupe amino, facultativement bloqué par un groupe protecteur, ou un sel de celui-ci, qui comprend la réaction d'un composé représenté par la formule (II) suivante:

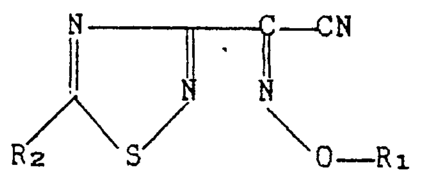

(II)

dans laquelle $R_1$ et $R_2$ ont la même signification que celle définie ci-dessus, ou un sel de celui-ci, avec un agent oxydant et une base, et ensuite l'élimination du groupe protecteur, si nécessaire.

2. Procédé tel que revendiqué dans la revendication 1, dans lequel la réaction est effectuée entre 0 °C et 70 °C.

3. Procédé tel que revendiqué dans la revendication 2, dans lequel la réaction est effectuée entre 15 °C et 50 °C.

4. Procédé tel que revendiqué dans la revendication 1, dans lequel l'agent oxydant est le peroxyde d'hydrogène ou l'oxygène.

5. Procédé tel que revendiqué dans la revendication 1, dans lequel la base est l'hydroxyde de sodium, l'hydroxyde de potassium, le carbonate de sodium, le carbonate de potassium, l'hydrogénocarbonate de sodium, l'hydrogénocarbonate de potassium, l'acétate de sodium ou l'acétate de potassium.

6. Procédé tel que revendiqué dans la revendication 5, dans lequel la base est l'hydroxyde de sodium.

7. Procédé tel que revendiqué dans la revendication 2, dans lequel le procédé est effectué dans un solvant aqueux.

8. Procédé tel que revendiqué dans la revendication 7, dans lequel le solvant est l'eau, un tampon ou un solvant composé d'un mélange d'eau ou d'un tampon et d'un alcool inférieur.

9. Procédé tel que revendiqué dans la revendication 8, dans lequel l'alcool inférieur est le méthanol ou l'éthanol.

**10.** Utilisation du dérivé de thiadiazolylacétamide de la revendication 1, comme intermédiaire pour l'élaboration de compositions antibactériennes.

**Patentansprüche**

**Patentanspruch für folgende Vertragsstaaten : BE, CH, DE, FR, GB, GR, IT, LI, LU, NL,**

**1.** Thiadiazolylacetamidderivat der Formel (I)

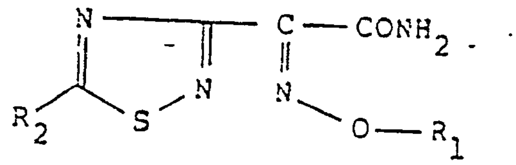

(I)

wobei $R_1$ eine Methylgruppe bedeutet und $R_2$ eine Aminogruppe darstellt, die gegebenenfalls durch eine Schutzgruppe blockiert ist, oder ein Salz davon.

**2.** Verbindung nach Anspruch 1, die 2-(5-Amino-1,2,4-thiadiazol-3-yl)-(Z)-2-methoxyiminoacetamid oder ein Salz davon ist.

**3.** Verbindung nach Anspruch 1, die 2-(5-Chloroacetamidol,2,4-thiadiazol-3-yl)-(Z)-2-methoxyiminoacetamidoder ein Salz davon ist.

**4.** Verfahren zur Herstellung eines Thiadiazolylacetamidderivates der Formel (I)

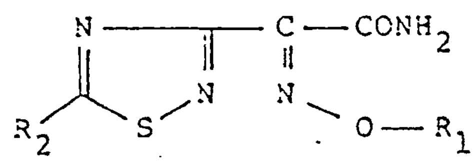

(I)

wobei $R_1$ eine Methylgruppe bedeutet und $R_2$ eine Aminogruppe darstellt, die gegebenenfalls durch eine Schutzgruppe blockiert ist, oder eines Salzes davon, das die Reaktion einer Verbindung der Formel (II)

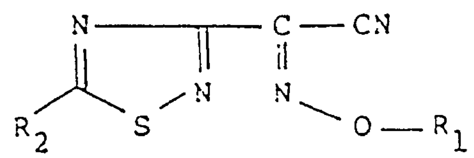

(II)

wobei $R_1$ und $R_2$ die gleichen Bedeutungen wie oben besitzen, oder eines Salzes davon mit einem Oxidationsmittel und einer Base und die anschliessende Entfernung der Schutzgruppe, falls notwendig, umfasst.

**5.** Verfahren nach Anspruch 4, wobei die Reaktion bei 0 bis 70 °C durchgeführt wird.

**6.** Verfahren nach Anspruch 5, wobei die Reaktion bei 15 bis 50 °C durchgeführt wird.

**7.** Verfahren nach Anspruch 4, wobei das Oxidationsmittel Wasserstoffperoxid oder Sauerstoff ist.

**8.** Verfahren nach Anspruch 4, wobei die Base Natriumhydroxid, Kaliumhydroxid, Natriumkarbonat, Kaliumkarbonat, Natriumhydrogenkarbonat, Kaliumhydrogenkarbonat, Natriumacetat oder Kaliumacetat ist.

**9.** Verfahren nach Anspruch 8, wobei die Base Natriumhydroxid ist.

**10.** Verfahren nach Anspruch 4, wobei das Verfahren in einem wässrigen Lösungsmittel durchgeführt wird.

**11.** Verfahren nach Anspruch 10, wobei das wässrige Lösungsmittel Wasser, ein Puffer oder ein Mischlösungsmittel aus Wasser oder einem Puffer und einem Niedrigalkohol ist.

**12.** Verfahren nach Anspruch 11, wobei der Niedrigalkohol Methanol oder Ethanol ist.

**13.** Verwendung eines Thiazolylacetamidderivates nach Anspruch 1 als Zwischenprodukt für die Herstellung von antibakteriellen Zusammensetzungen.

**Patentansprüche für folgenden Vertragsstaaten : AT, ES**

**1.** Verfahren zur Herstellung eines Thiadiazolylacetamidderivates, dargestellt durch die Formel (I)

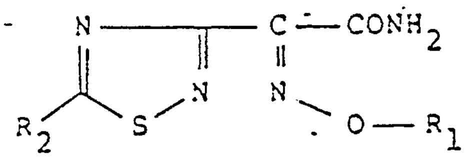

(I)

wobei $R_1$ eine Methylgruppe bedeutet und $R_2$ eine Aminogruppe darstellt, die gegebenenfalls durch eine Schutzgruppe blockiert ist, oder eines Salzes davon, das die Reaktion einer Verbindung der Formel (II)

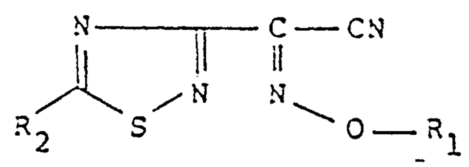

(II)

wobei $R_1$ und $R_2$ die gleichen Bedeutungen wie oben besitzen, oder eines Salzes davon mit einem Oxidationsmittel und einer Base und anschliessend das Entfernen der Schutzgruppe, falls notwendig, umfasst.

**2.** Verfahren nach Anspruch 1, wobei die Reaktion bei 0 bis 70 °C durchgeführt wird.

**3.** Verfahren nach Anspruch 2, wobei die Reaktion bei 15 bis 50 °C durchgeführt wird.

**4.** Verfahren nach Anspruch 1, wobei das Oxidationsmittel Wasserstoffperoxid oder Sauerstoff ist.

**5.** Verfahren nach Anspruch 1, wobei die Base Natriumhydroxid, Kaliumhydroxid, Natriumkarbonat, Kaliumkarbonat, Natriumhydrogenkarbonat, Kaliumhydrogenkarbonat, Natriumacetat oder Kaliumacetat ist.

**6.** Verfahren nach Anspruch 5, wobei die Base Natriumhydroxid ist.

**7.** Verfahren nach Anspruch 2, wobei das Verfahren in einem wässrigen Lösungsmittel durchgeführt wird.

**8.** Verfahren nach Anspruch 7, wobei das wässrige Lösungsmittel Wasser, ein Puffer oder ein Mischlösungsmittel aus Wasser oder einem Puffer und einem Niedrigalkohol ist.

**9.** Verfahren nach Anspruch 8, wobei der Niedrigalkohol Methanol oder Ethanol ist.

**10.** Verwendung eines Thiadiazolylacetamidderivates nach Anspruch 1 als Zwischenprodukt für die Herstellung von antibakteriellen Zusammensetzungen.